# DEMANDE DE BREVET EUROPEEN

(11) **EP 0 714 970 A1**
(43) Date de publication de la demande: **05.06.1996**
(21) Numéro de dépôt: 95402620.9
(22) Date de dépôt: 21.11.1995
(51) Int. Cl.: C10M 135/04, B01J 37/20, C07C 319/00

(54) **Hydrocarbures éthyléniques sulfurés par le soufre élémentaire en présence d'hydroxydes de métaux alcalins ou alcalino-terreux et en présence de glycols, polyglycols ou de leurs éthers alkyliques et/ou en présence d'eau**

(30) Priorité: 28.11.1994 FR 9414210; 28.11.1994 FR 9414208
(71) Demandeur: INSTITUT FRANCAIS DU PETROLE, F-92502 Rueil-Malmaison (FR)
(72) Inventeur: Born, Maurice, F-92000 Nanterre (FR); Delfort, Bruno, F-75005 Paris (FR); Lacome, Thierry, F-92500 Rueil Malmaison (FR)

(57) **Abrégé**

On décrit des produits soufrés dérivant d'hydrocarbures mono ou polyethylèniques par sulfuration au moyen de soufre élémentaire en présence d'au moins hydroxyde de métal alcalin ou alcalino -terreux et en présence d'au moins un glycol, polyglycol ou monoéther de glycol ou de polyglycol,et/ou en présence d'eau.

Les produits soufrés, qui peuvent contenir de 20 à 70 % en masse de soufre, sont généralement solubles dans les lubrifiants, dont ils améliorent les propriétés antiusure et extrême-pression.

Ils peuvent également être utilisés comme agents de sulfuration notamment dans la préparation de catalyseurs de raffinage de produits pétroliers.

## Description

L'invention concerne de nouveaux produits organiques soufrés, un procédé pour leur préparation et leurs utilisations.

Divers produits organiques soufrés sont utilisés depuis longtemps comme additifs dans les lubrifiants pour en améliorer notamment les propriétés antiusure et extrême-pression. Il peut s'agir notamment de lubrifiants pour moteurs, pour engrenages fortement chargés ou de lubrifiants pour le travail des métaux.

Comme additifs antiusure et extrême-pression, ont été largement décrits des produits organiques soufrés obtenus par divers procédés comprenant principalement deux étapes :
1) la formation d'un produit d'addition entre un chlorure de soufre (mono ou di-chlorure de soufre) et un composé à insaturation éthylènique, et
2) la réaction de ce produit d'addition avec un composé sulfuré: sulfure, hydrosulfure ou polysulfure de métal alcalin (par exemple le sodium), mis en jeu tels quels ou formés in situ par réaction d'hydrogène sulfuré (H₂S) ou de mercaptans avec un hydroxyde de métal alcalin (par exemple la soude). Pour accroître la teneur en soufre des produits obtenus, il était possible de mettre en jeu du soufre élémentaire au cours de la seconde étape décrite ci-dessus.

Du fait de la mise en jeu de composés chlorés dans la première étape de leur préparation, les produits soufrés de ce type contiennent toujours une certaine quantité résiduelle de chlore, qui a pu être réduite à moins de 250 ppm par diverses améliorations apportées au procédé, notamment par la demanderesse, ces dernières années. De plus, ces procédés engendrent la formation, comme sous produits, de chlorure de métal alcalin (en général NaCl) et de dérivés organiques alcalins hydrosolubles, que l'on retrouve sous la forme d'une solution aqueuse très polluante, produite en quantité très importante et, et de ce fait très coûteuse à éliminer.

On connaît par ailleurs divers procédés de préparation de produits soufrés ne mettant pas en jeu de composés chlorés. Certains de ces procédés sont basés sur la sulfuration des oléfines par le soufre élémentaire et l'hydrogène sulfuré (H2S), ces procédés présentent l'inconvénient de mettre en jeu un réactif extrêmement toxique, difficile à stocker et à transporter et de développer des pressions généralement élevées qui peuvent aller par exemple jusqu'à 90 bars.

D'autres procédés mettent en jeu du soufre élémentaire et des hydrosulfures alcalins ou alcalino-terreux.

On a maintenant découvert qu'il était possible de préparer des produits soufrés à partir d'hydrocarbures mono ou polyéthylèniques en utilisant du soufre élémentaire comme seul réactif soufré, en opérant en présence d'au moins un hydroxyde de métal alcalin ou alcalino-terreux et en présence d'au moins un glycol ou polyglycol ou monoéther de glycol ou de polyglycol, et/ou d'eau.

Les produits soufrés de l'invention sont exempts de chlore, ils présentent en général une teneur en soufre pouvant aller d'environ 20 à 70% en masse selon l'hydrocarbure éthylènique de départ.

Les produits soufrés de l'invention sont solubles à des degrés divers dans les huiles minérales et dans les polyalphaoléfines hydrogénées, même - ce qui est tout à fait inattendu - les produits ayant une teneure en soufre élevée.

Les produits soufrés de l'invention peuvent être définis d'une manière générale par le fait qu'ils sont obtenus par réaction d'au moins un hydrocarbure mono ou polyéthylènique renfermant en général de 2 à 36 atomes de carbone et de 1 à 3 insaturations éthylèniques avec une quantité appropriée de soufre élémentaire en présence d'un hydroxyde de métal alcalin ou alcalino-terreux et en présence d'au moins un alkylèneglycol ou polyalkylèneglycol de masse moléculaire allant jusqu'à environ 200, ou d'un dérivé monoéther de ceux-ci, et/ou en présence d'eau.

Les hydrocarbures mono ou polyéthyléniques de départ peuvent être à chaîne ouverte, linéaire ou ramifiée, ou cycliques. Comme exemple, on peut citer : l'éthylène, le propylène, le butène-1, les n-butènes-2, l'isobutène, les divers pentènes, les divers hexènes (par exemple le 2, 3 -diméthyl 1- butène) ainsi que le 1, 3-butadiène, l'isoprène, le cyclopentadiène, le dicyclopentadiène, les dimères et trimères de l'isobutène, les trimères et tétramères du propylène ainsi que les polyalphaoléfines non hydrogénées de faible masse molaire (allant par exemple jusqu'à environ 500). On utilise de préférence l'isobutène.

L'hydroxyde de métal alcalin ou alcalino-terreux mise en jeu peut-être choisi parmi la soude, la potasse, la lithine et les hydroxydes de calcium (chaux) ou de baryum (baryte). On utilise le plus souvent la soude ou la potasse.

La proportion d'hydroxyde de métal alcalin ou alcalino-terreux mis en jeu peut aller de 310⁻³ à 1,2 par insaturation éthylénique de l'hydrocarbure de départ.

On remarque avec surprise que l'hydroxyde de métal alcalin ou alcalino-terreux peut-être utilisé notamment en très faible proportion (quasi catalytique) et que les produits soufrés finalement obtenus ne renferment pas de métaux alcalins ou alcalino-terreux et ne présentent aucun caractère basique. Aussi, peut on penser que l'hydroxyde agit essentiellement comme promoteur de la réaction de sulfuration.

Lorsque l'hydroxyde de métal alcalin ou alcalino-terreux est mis en jeu essentiellement en présence d'eau, la proportion d'eau peut varier par exemple de 50 à 500 cm3 par insaturation éthylénique de l'hydrocarbure de départ. Dans ce cas, on met en jeu une proportion de soufre élémentaire allant jusqu'à environ 250 g (soit environ 8 moles) par insaturation éthylénique de l'hydrocarbure de départ.

Dans le cadre de l'invention, on peut également mettre en jeu au moins un glycol, polyglycol et/ou dérivé monoéther alkylique de glycol ou de polyglycol. Dans ce cas, la proportion d'hydroxyde de métal alcalin ou alcalino-terreux mise en jeu est généralement de 0,004 à 1 mole par insaturation éthylénique de l'hydrocarbure à sulfurer.

Les glycols, polyglycols et leurs dérivés monoéthers que l'on peut mettre en jeu avec l'hydroxyde de métal alcalin ou alcalino-terreux sont choisis parmi l'éthylèneglycol, le propylèneglycol , les di,-tri-et tétraéthylèneglycols, les di,-tri et tétrapropylèneglycols et leurs dérivés monoéthers alkyliques dont le groupe alkyle renferme de 1 à 30 atomes de carbone. Ces composés peuvent être utilisés seuls ou en mélanges. On utilise de préférence l'éthylèneglycol, le propylèneglycol et leur monométhyléthers.

La proportion de glycol ou de dérivé de glycol mise en jeu peut varier dans de larges limites. Elle peut être plus particulièrement d'environ 10 à 150 g par insaturation de l'hydrocarbure mono-ou polyéthylénique de départ. Dans ce cas, on met en jeu une proportion de soufre élémentaire allant jusqu'à environ 200 g (soit environ 6 moles) par insaturation éthylénique dans l'hydrocarbure de départ.

Dans le cas où, conjointement au glycol, polyglycol ou éther monoalkylique, l'on met en jeu de l'eau dans le milieu réactionnel, la proportion peut aller par exemple jusqu'à 20 g par insaturation éthylénique de l'hydrocarbure de départ.

En général, la réaction est réalisée à une température de 50 à 200°C et le plus souvent de 90 à160°C.

La pression, qui dépend de manière prépondérante de la nature du ou des hydrocarbures mono ou polyéthylèniques de départ, peut aller de la pression atmosphérique jusqu'à par exemple 50 bars. Ainsi, pour les hydrocarbures éthyléniques gazeux dans les conditions normales, la pression s'établit entre 10 et 50 bars selon le proportion entre l'hydrocarbure éthylénique et l'hydroxyde de métal alcalin ou alcalino-terreux mis en jeu.

La durée de la réaction est par exemple de 0,5 à 24 heures .

Les produits de l'invention sont des produits liquides renfermant généralement de 20 à 70 % en masse de soufre ; ils sont limpides et homogènes même aux hautes teneurs en soufre. Leur coloration varie, selon la nature de l'hydrocarbure de départ et la quantité de soufre fixée. Ils peuvent être de très peu ou peu colorés (c'est la cas par exemple pour les hydrocarbures monoéthyléniques tels que l'isobutène) à très colorés (dans le cas par exemple des hydrocarbures polyéthyléniques).

Dans le cas des hydrocarbures monoéthyléniques, les produits soufrés se caractérisent par la quasi absence d'atomes de carbone éthyléniques.

Les teneurs en soufre des produits de l'invention leur confèrent des propriétés anti usure et extrême - pression telles qu'ils peuvent être utilisés avantageusement comme additifs dans les huiles et graisses lubrifiantes, notamment dans la formulation d'huiles pour engrenages d'automobiles et d'huiles industrielles.

Pour ces utilisations, on incorpore en général les produits de l'invention aux compositions lubrifiantes à des concentrations de 0,05 à 20 %, de préférence de 0,5 à 10 % en masse.

Certains des produits soufrés de l'invention, notamment ceux qui dérivent de la sulfuration de l'isobutène, peuvent encore être utilisés comme agents de sulfuration en particulier dans la préparation de catalyseurs de raffinage de produits pétroliers.

Les exemples suivants illustrent l'invention.

### Exemple 1

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 50g (1,56 moles) de soufre, 15g (0,375 mole) de soude, 135g d'eau et 72g (1,285 moles) d'isobutène. On porte le milieu à la température de 130°C pendant 8 heures. La pression maximale atteinte est de 33 bars. Après retour à la température ambiante, on extrait le milieu avec 150 ml de n-heptane. Après lavage à la soude à 10% en masse, puis lavage à l'eau, on évapore le n-heptane sous pression réduite et on recueille 75g d'un liquide limpide dont la teneur en soufre est de 43,8% en masse.

### Exemple 2

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 75g (2,34 moles) de soufre, 15g (0,375 mole) de soude, 135g d'eau et 72g (1,285 moles) d'isobutène. On porte le milieu à la température de 130°C pendant 8 heures. La pression maximale atteinte est de 30 bars. Après retour à la température ambiante, on extrait le milieu avec 150 ml de n-heptane. Après lavage à la soude à 10% en masse, puis lavage à l'eau, on évapore le n-heptane sous pression réduite et on recueille 103g d'un liquide limpide dont la teneur en soufre est de 55,4% en masse.

### Exemple 3

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 112,5g (3,51 moles) de soufre, 22,5 g (0,562 mole) de soude, 202g d'eau et 72g (1,285 moles) d'isobutène. On porte le milieu à la température de 130°C pendant 10 heures. La pression maximale atteinte est de 32 bars. Après retour à la température ambiante, on extrait le milieu avec 150 ml de n-heptane. Après lavage à la soude à 10% en masse, puis lavage à l'eau, on évapore le n-heptane sous pression réduite et on recueille 154g d'un liquide limpide dont la teneur en soufre est de 57,4% en masse.

### Exemple 4

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 35,7g (1,11 moles) de soufre, 7,5g (0,187 mole) de soude, 68g d'eau et 72g (1,285 moles) d'isobutène. On porte le milieu à la température de 130°C pendant 8 heures. La pression maximale atteinte est de 32 bars. Après retour à la température ambiante, on extrait le milieu avec 150 ml de n-heptane. Après lavage à la soude à 10% en masse, puis lavage à l'eau, on évapore le n-heptane sous pression réduite et on recueille 54g d'un liquide limpide dont la teneur en soufre est de 55,9% en masse.

### Exemple 5

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 225g (7,03 moles) de soufre, 45g (1,125 moles) de soude, 405g d'eau et 72g (1,285 moles) d'isobutène. On porte le milieu à la température de 130°C pendant 10 heures. La pression maximale atteinte est de 32 bars. Après retour à la température ambiante, on extrait le milieu avec 150 ml de toluène. Après lavage à la soude à 10% en masse, puis lavage à l'eau, on évapore le toluène sous pression réduite et on recueille 213g d'un liquide limpide dont la teneur en soufre est de 64,5% en masse.

### Exemple 6

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 300g (9,37 moles) de soufre, 60g (1,50 moles) de soude, 540g d'eau et 72g (1,285 moles) d'isobutène. On porte le milieu à la température de 130°C pendant 12 heures. La pression maximale atteinte est de 35 bars. Après retour à la température ambiante, on extrait le milieu avec 150 ml de toluène. Après lavage à la soude à 10% en masse, puis lavage à l'eau, on évapore le toluène sous pression réduite et on recueille 232g d'un liquide limpide dont la teneur en soufre est de 67,4% en masse.

### Exemple 7

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 75g (2,34 moles) de soufre, 7,5g (0,187 mole) de soude, 135g d'eau et 72g (1,285 moles) d'isobutène. On porte le milieu à la température de 130°C pendant 8 heures. La pression maximale atteinte est de 31 bars. Après retour à la température ambiante, on extrait le milieu avec 150 ml de toluène. Après lavage à la soude à 10% en masse, puis lavage à l'eau, on évapore le toluène sous pression réduite et on recueille 86g d'un liquide limpide dont la teneur en soufre est de 63% en masse.

### Exemple 8

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 75g (2,34 moles) de soufre, 30g (0,75 mole) de soude, 135g d'eau et 72g (1,285 moles) d'isobutène. On porte le milieu à la température de 130°C pendant 10 heures. La pression maximale atteinte est de 32 bars. Après retour à la température ambiante, on extrait le milieu avec 150 ml de n-heptane. Après lavage à la soude à 10% en masse, puis lavage à l'eau, on évapore le n-heptane sous pression réduite et on recueille 68 g d'un liquide limpide dont la teneur en soufre est de 56,8% en masse.

### Exemple 9

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 112,5g (3,52 moles) de soufre, 0,2 g (0,005 mole) de soude, 400g d'eau et 72g (1,285 moles) d'isobutène. On porte le milieu à la température de 145°C pendant 12 heures. La pression maximale atteinte est de 36 bars. Après retour à la température ambiante, on extrait le milieu avec 150 ml de n-heptane. Après lavage à la soude à 10% en masse, puis lavage à l'eau, on évapore le n-heptane sous pression réduite et on recueille 100g d'un liquide limpide dont la teneur en soufre est de 61,5% en masse.

### Exemple 10

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 225g (7,03 moles) de soufre, 0,2g (0,005 mole) de soude, 400g d'eau et 72g (1,285 moles) d'isobutène. On porte le milieu à la température de 140°C pendant 12 heures. La pression maximale atteinte est de 38 bars. Après retour à la température ambiante, on extrait le milieu avec 150 ml de toluène. Après lavage à la soude à 10% en masse, puis lavage à l'eau, on évapore le toluène sous pression réduite et on recueille 90g d'un liquide limpide dont la teneur en soufre est de 67,0 % en masse.

### Exemple 11

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 35g (1,09 moles) de soufre, 15g (0,375 mole) de soude, 135g d'eau et 70g (0,83 mole) de 2,3-diméthylbutène-1. On porte le milieu à la température de 130°C pendant 10 heures. La pression maximale atteinte est de 10 bars. Après retour à la température ambiante, on extrait le milieu avec 150 ml de n-heptane. Après lavage à la soude à 10% en masse, puis lavage à l'eau, on évapore le n-heptane sous pression réduite et on recueille 40g d'un liquide limpide dont la teneur en soufre est de 39,3% en masse.

### Exemple 12

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 39,5g (1,23 moles) de soufre, 15g (0,375 mole) de soude, 135g d'eau et 75g (0,567 mole) de dicyclopentadiène. On porte le milieu à la température de 130°C pendant 12 heures. La pression maximale atteinte est de 7 bars. Après retour à la température ambiante, on extrait le milieu avec 150 ml de toluène. Après lavage à la soude à 10% en masse, puis lavage à l'eau, on évapore le toluène sous pression réduite et on recueille 82g d'un liquide limpide dont la teneur en soufre est de 20,9 % en masse.

### Exemple 13

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 50g (1,56 moles) de soufre, 15g (0,375 mole) de soude, 135g d'eau, 67g (1,196 moles) d'isobutène et 3,6g (0,064 mole) de 1,3-butadiène. On porte le milieu à la température de 130°C pendant 12 heures. La pression maximale atteinte est de 30 bars. Après retour à la température ambiante, on extrait le milieu avec 150 ml de n-heptane. Après lavage à la soude à 10% en masse, puis lavage à l'eau, on évapore le n-heptane sous pression réduite et on recueille 55g d'un liquide limpide dont la teneur en soufre est de 51,9% en masse.

### Exemple 14

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 76,9g (2,40 moles) de soufre, 15g (0,375 mole) de soude, 135g d'eau, 36,5g (0,652 mole) d'isobutène et 46,7g (0,35 mole) de dicyclopentadiène. On porte le milieu à la température de 130°C pendant 10 heures. La pression maximale atteinte est de 21 bars. Après retour à la température ambiante, on extrait le milieu avec 150 ml de toluène. Après lavage à la soude à 10% en masse, puis lavage à l'eau, on évapore le toluène sous pression réduite et on recueille 126g d'un liquide limpide dont la teneur en soufre est de 42,5% en masse.

### Exemple 15

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 112,5g (3,51 moles) de soufre, 0,2g (0,0036 mole) de potasse, 400g d'eau et 72g (1,285 moles) d'isobutène. On porte le milieu à la température de 130°C pendant 12 heures. La pression maximale atteinte est de 39 bars. Après retour à la température ambiante, on extrait le milieu avec 150 ml de n-heptane. Après lavage à la soude à 10% en masse, puis lavage à l'eau, on évapore le n-heptane sous pression réduite et on recueille 130g d'un liquide limpide dont la teneur en soufre est de 64,5% en masse.

L'examen par C13 RMN et par analyse élémentaire des produits préparés dans les exemples de 1 à 11 et 15 indique l'absence d'atomes de carbone éthylénique et l'absence de sodium pour tous les produits des exemples de 1 à 15.

### Exemple 16: Examen de la solubilité des produits de l'invention

Les produits de l'invention préparés comme décrit dans les exemples 1 à 11 sont examinés pour leur solubilité dans une huile minérale 130 Neutral Solvent et dans une huile synthétique polyalphaoléfine hydrogénée (PAO 6), à une concentration permettant d'ajuster la teneur en soufre à 2% en masse. Les résultats sont rassemblés dans le tableau I.

### Exemple 17: Caractérisation des propriétés extrême-pression

Les produits de l'invention préparés comme décrit dans les exemples 1, 2, 3, 5, 6 et 7 ci-dessus sont caractérisés pour leurs propriétés extrême-pression dans une huile minérale 130 Neutral Solvent à une concentration permettant d'ajuster la concentration en soufre due à l'additif à 2% en masse. La caractérisation est effectuée au moyen d'une machine 4 billes selon l'essai ASTM D2783. Les résultats sont rassemblés dans le tableau II.

### Exemple 18

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 75 g (2,34 moles) de soufre, 15 g (0,375 mole) de soude, 15 g d'eau, 150 ml d'éthylèneglycol et 72 g (1,285 moles) d'isobutène. On porte le milieu à la température de 130°C pendant 9 heures. La pression maximale atteinte est de 28 bars. Après retour à la température ambiante, on extrait le milieu avec 150 ml de n-heptane. Après lavage à la soude à 10 % en masse, puis lavage à l'eau, on évapore le n-heptane sous pression réduite et on recueille 93 g d'un liquide limpide dont la teneur en soufre est de 48,4 % en masse.

### Exemple 19

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 75 g (2,34 moles) de soufre, 15 g (0,375 mole) de soude, 15 g d'eau, 150 ml de propylèneglycol et 72 g (1,285 moles) d'isobutène. On porte le milieu à la température de 130°C pendant 12 heures. La pression maximale atteinte est de 30 bars. Après retour à la température ambiante, on extrait le milieu avec 150 ml de n-heptane. Après lavage à la soude à 10 % en masse puis lavage à l'eau, on évapore le n-heptane sous pression réduite et on recueille 102 g d'un liquide limpide dont la teneur en soufre est de 48,4 % en masse.

### Exemple 20

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 150 g (4,68 moles) de soufre, 30 g (0,75 mole) de soude, 150 ml d'éthylèneglycol et 72 g (1,285 moles) d'isobutène. On porte le milieu à la température de 130°C pendant 10 heures. La pression maximale atteinte est de 31 bars. Après retour à la température ambiante, on extrait le milieu avec 150 ml de n-heptane. Après lavage à la soude à 10 % en masse, puis lavage à l'eau, on évapore le n-heptane sous pression réduite et on recueille 164 g d'un liquide limpide dont la teneur en soufre est de 57,2 % en masse.

### Exemple 21

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 150 g (4,68 moles) de soufre, 0,2 g (0,75 mole) de soude, 150 ml d'éthylèneglycol et 72 g (1,285 moles) d'isobutène. On porte le milieu à la température de 130°C pendant 12 heures. La pression maximale atteinte est de 31 bars. Après retour à la température ambiante, on extrait le milieu avec 150 ml de n-heptane. Après lavage à la soude à 10 % en masse, puis lavage à l'eau, on évapore le n-heptane sous pression réduite et on recueille 34 g d'un liquide limpide dont la teneur en soufre est de 60,3 % en masse.

### Exemple 22

Dans un réacteur de 1 litre permettant d'opérer sous pression, équipé d'un agitateur, d'un système d'introduction de réactifs liquides ou gazeux ainsi que d'un système de contrôle de la température et de la pression, on introduit 75 g (2,34 moles) de soufre, 15 g (0,375 mole) de soude, 150 ml d'éthylèneglycol et 67,1 g (1,2 moles) d'isobutène et 3,6 g (0,064 mole) de 1,3-butadiène. On porte le milieu à la température de 130°C pendant 10 heures. La pression maximale atteinte est de 25 bars. Après retour à la température ambiante, on extrait le milieu avec 150 ml de n-heptane. Après lavage à la soude à 10 % en masse, puis lavage à l'eau, on évapore le n-heptane sous pression réduite et on recueille 34 g d'un liquide limpide dont la teneur en soufre est de 50,6 % en masse.

L'examen par C13 RMN et par analyse élémentaire des produits préparés dans les exemples de 18 à 21 indique l'absence d'atomes de carbone éthyléniques, et l'absence de sodium pour tous les produits des exemples de 18 à 22.

### Exemple 23 : Examen de la solubilité des produits de l'invention

Les produits de l'invention préparés comme décrit dans les exemples 18 à 22 ci-dessus sont examinés pour leur solubilité dans une huile minérale 130 Neutral Solvent et une huile synthétique polyalphaoléfine hydrogénée (PAO 6) à une concentration permettant d'ajuster la teneur en soufre à 2 % en masse. Les résultats sont rassemblés dans le tableau III.

### Exemple 24 : Caractérisation des propriétés extrême-pression

Les produits de l'invention préparés comme décrit dans les exemples 18 à 21 ci-dessus sont caractérisés pour leur propriétés extrême-pression dans une huile minérale 130 Neutral Solvent à une concentration permettant d'ajuster la teneur en soufre due à l'additif à 2 % en masse. La caractérisation est effectuée au moyen d'une machine 4 billes selon l'essai ASTM D2783. Les résultats sont rassemblés dans le tableau IV.

**TABLEAU I**

| PRODUIT DE L'EXEMPLE N° | % en masse ADDITIF/HUILE | % en masse S/HUILE | SOLUBILITE/HUILE MINERALE 130N | SOLUBILITE/HUILE SYNTHETIQUE PAO 6 |
|---|---|---|---|---|
| 1 | 4,56 | 2 | oui | oui |
| 2 | 3,61 | 2 | oui | oui |
| 3 | 3,48 | 2 | oui | oui |
| 4 | 3,57 | 2 | oui | oui |
| 5 | 3,1 | 2 | oui | oui |
| 6 | 2,96 | 2 | oui | oui |
| 7 | 3,17 | 2 | oui | oui |
| 8 | 3,52 | 2 | oui | oui |
| 9 | 3,25 | 2 | oui | oui |
| 10 | 2,98 | 2 | oui | oui |
| 11 | 5,08 | 2 | oui | oui |

**TABLEAU II**

| PRODUIT DE L'EXEMPLE N° | % en masse ADDITIF/HUILE | % en masse S/HUILE | Essais sur machine 4 billes | |
|---|---|---|---|---|
| | | | Charge de soudure (daN) | Indice charge/usure (daN) |
| 1 | 4,56 | 2 | 380 | 71 |
| 2 | 3,61 | 2 | 430 | 80 |
| 3 | 3,48 | 2 | 410 | 79 |
| 5 | 3,1 | 2 | 400 | 77 |
| 6 | 2,96 | 2 | 390 | 72 |
| 7 | 3,17 | 2 | 440 | 84 |

**TABLEAU III**

| PRODUIT DE | % MASSE | % MASSE | SOLUBILITE | SOLUBILITE/ |
|---|---|---|---|---|
| L'EXEMPLE | ADDITIF/ | S/ | HUILE MINERALE | HUILE SYNTHETIQUE |
| N° | HUILE | HUILE | 130N | PAO 6 |
| | | | | |
| 18 | 4,13 | 2 | oui | oui |
| 19 | 4,13 | 2 | oui | oui |
| 20 | 3,49 | 2 | oui | oui |
| 21 | 3,32 | 2 | oui | non |
| 22 | 3,95 | 2 | oui | oui |

**TABLEAU IV**

| PRODUIT DE L'EXEMPLE N° | % ADDITIF/HUILE | % S/HUILE | Essais sur machine 4 billes | |
|---|---|---|---|---|
| | | | Charge de soudure (daN) | Indice charge/usure (daN) |
| 18 | 4,13 | 2 | 410 | 80 |
| 19 | 4,13 | 2 | 400 | 74 |
| 20 | 3,49 | 2 | 390 | 72 |
| 21 | 3,32 | 2 | 420 | 81 |

## Revendications

1. Produit soufré caractérisé en ce qu'il est obtenu par réaction d'au moins un hydrocarbure mono ou polyéthylènique renfermant de 2 à 36 atomes de carbone et de 1 à 3 insaturations éthyléniques avec du soufre élémentaire en présence d'au moins un hydroxyde de métal alcalin ou alcalino-terreux et en présence d'au moins un glycol, polyglycol de masse molaire allant jusqu'à environ 200, ou d'un de leurs dérivés éther alkylique et/ou en présence d'eau.

2. Produit soufré selon la revendication 1, caractérisé en ce que l'hydrocarbure mono ou polyéthylénique est choisi parmi l'éthylène, le propylène, le butène-1, les n-butènes-2, l'isobutène, les divers pentènes, les divers héxènes ainsi que le 1, 3-butadiène, l'isoprène, le cyclopentadiène, le dicyclopentadiène, les dimères et trimères de l'isobutène, les trimères et tétramères du propylène, ainsi que les polyalphaoléfines non hydrogénées de masse molaire allant jusqu'à environ 500.

3. Produit soufré selon la revendication 1 ou 2, caractérisé en ce que ledit hydrocarbure est l'isobutène.

4. Produit soufré selon l'une des revendications 1 à 3, caractérisé en ce que l'on met en jeu de l'eau et au moins un hydroxyde de métal alcalin choisi parmi la soude, le potasse, le lithine, la chaux et la baryte, utilisé en une proportion de 3.10⁻³ à 1,2 moles par insaturation éthylènique de l'hydrocarbure de départ.

5. Produit soufré selon l'une des revendications 1 à 4, caractérisé en ce que l'on met en jeu une proportion d'eau de 50 à 500 cm3 par insaturation éthylènique de l'hydrocarbure de départ.

6. Produit soufré selon l'une des revendications 1 à 5, caractérisé en ce que l'on met en jeu une proportion de soufre élémentaire allant jusqu'à environ 250 g (soit environ 8 moles) par insaturation éthylènique de l'hydrocarbure de départ.

7. Produit soufré selon l'une des revendications 1 à 3, caractérisé en ce que l'on met en jeu un glycol, un polyglycol ou un dérivé éther alkylique de glycol ou de polyglycol et au moins un hydroxyde de métal alcalin choisi parmi la soude, la potasse, la lithine, la chaux et la baryte, utilisé en une proportion de 0,004 à 1 mole par insaturation éthylènique de l'hydrocarbure de départ.

8. Produit soufré selon la revendication 7, caractérisé en ce que le glycol, polyglycol ou dérivé monoéther de glycol ou de polyglycol est choisi parmi l'éthylèneglycol, le propylèneglycol, les di-, tri- et tétraéthylèneglycols, les di-, tri, et tétrapropylèneglycols, et leurs dérivés mono-éthers alkyliques dont le groupe alkyle renferme de 1 à 30 atomes de carbone.

9. Produit soufré selon la revendications 5, caractérisé en ce que l'on met en jeu de l'éthylèneglycol, du propylèneglycol, mono-méthylethers.

10. Produit soufré selon l'une des revendications 7 à 9, caractérisé en ce que le glycol, polyglycol ou monoéther de glycol est mis en jeu en une proportion d'environ 10 à 150 g par insaturation éthylènique de l'hydrocarbure de départ.

11. Produit soufré selon l'une des revendications 7 à 10, caractérisé en ce que l'on met en jeu une proportion de soufre élémentaire allant jusqu'à environ 200 g (soit 6 moles) par insaturation éthylènique dans l'hydrocarbure de départ.

12. Produit soufré selon l'une des revendications 7 à 11, caractérisé en ce que l'on met en jeu en outre de l'eau en une proportion allant jusqu'à 20 g par insaturation éthylènique de l'hydrocarbure de départ.

13. Produit soufré selon l'une des revendications 1 à 12, caractérisé en ce que la réaction est réalisée à une température de 50 à 200°C et sous une pression allant de la pression atmosphérique jusqu'à 50 bars.

14. Produit soufré selon l'une des revendications 1 à 13, caractérisé en ce qu'il renferme de 20 à70 % en masse de soufre.

15. Utilisation d'un produit soufré selon l'une des revendications 1à 14 comme additif dans des compositions lubrifiantes, à une concentration de 0,05 à 20 % en masse.

16. Utilisation d'un produit soufré selon l'une des revendications 1 à 15 comme agent de sulfuration dans la préparation de catalyseurs de raffinage de produits pétroliers.
